# EUROPÄISCHE PATENTANMELDUNG

(11) **EP 3 242 396 A1**
(43) Veröffentlichungstag der Anmeldung: **08.11.2017**
(21) Anmeldenummer: 16168126.7
(22) Anmeldetag: 03.05.2016
(51) Int. Cl.: H03H 7/42, G01R 33/36

(54) **VORRICHTUNG ZUR ÜBERTRAGUNG VON MAGNETRESONANZSIGNALEN MITTELS DIFFERENTIELLER LEITUNGSFÜHRUNG**

(71) Anmelder: Siemens Healthcare GmbH, 91052 Erlangen (DE)
(72) Erfinder: Biber, Stephan, Dr., 91056 Erlangen (DE); Bollenbeck, Jan, 91330 Eggolsheim (DE); Hemmerlein, Martin, 96052 Bamberg (DE)

(57) **Zusammenfassung**

Die Erfindung betrifft eine Übertragungsvorrichtung (70) zum Übertragen eines Zwischenfrequenzsignals und eines Oszillatorsignals zum Heruntermischen des Zwischenfrequenzsignals, sowie einen Magnetresonanztomographen mit einer Lokalspule (50), einer Empfangseinheit (22) und der Übertragungsvorrichtung (70). Die Übertragungsvorrichtung (70) weist eine symmetrische Übertragungsleitung (72) zur Übertragung des Oszillatorsignals und des Zwischenfrequenzsignals und ein Symmetrierglied (71) zur Anpassung einer unsymmetrischen Signalquelle und/oder Signalsenke an die symmetrische Übertragungsleitung (70) auf. Das Symmetrierglied (71) weist nur Ferrit-freie Induktivitäten auf. Die Lokalspule (50) und die Empfangseinheit (22) stehen über die Übertragunsvorrichtung (70) in Signalverbindung.

## Beschreibung

Die Erfindung betrifft eine Übertragungsvorrichtung zum Übertragen eines Zwischenfrequenzsignals und eines Oszillatorsignals zum Heruntermischen des Hochfrequenz-Empfangssignals in eine Zwischenfrequenzebene sowie einen Magnetresonanztomographen mit einer erfindungsgemäßen Übertragungsvorrichtung.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, auch als Magnetresonanzsignal bezeichnet, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt.

Zur Anregung der Präzession der Spins sind magnetische Wechselfelder mit einer Frequenz, die der Larmorfrequenz bei der jeweiligen statischen Magnetfeldstärke entspricht, und sehr hohen Feldstärken bzw. Leistungen erforderlich. Zur Verbesserung des Signal-Rauschverhältnisses des von den Antennen empfangenen Magnetresonanzsignals werden häufig als Lokalspulen bezeichnete Antennen verwendet, die unmittelbar am Patienten angeordnet werden.

Zur Bildgebung müssen die von der Lokalspule empfangenen Magnetresonanzsignale zu einer Empfangseinrichtung des Magnetresonanztomographen übertragen werden. Häufig werden die Magnetresonanzsignale auch durch Mischen in einen niedrigeren Frequenzbereich (Zwischenfrequenz) heruntergesetzt. Um die Phasen- und Frequenz-Merkmale des ursprünglichen Magnetresonanzsignals bei der Auswertung erfassen zu können, ist dann ebenfalls als Referenz das Mischsignal bzw. Oszillatorsignal erforderlich und zu übertragen.

Das Magnetresonanzsignal weist üblicherweise eine Bandbreite von einem Megahertz auf. Nachdem es auf eine Zwischenfrequenz von beispielsweise 10 MHz heruntergemischt wurde, handelt es sich bei dem Zwischenfrequenzsignal folglich um ein relativ breitbandiges Signal.

Zur Übertragung der Signale werden üblicherweise Koaxialkabel verwendet, die insbesondere in dünnen und flexiblen Ausführungsformen teuer und schwierig zu verarbeiten sind.

Aus der Druckschrift DE 10104260 A1 ist ein Symmetrierglied für zwei Frequenzen bekannt. Die Druckschrift DE 102013209450 A1 beschreibt ein Symmetrierglied für einen verbreiterten Frequenzbereich.

Es ist daher die Aufgabe der Erfindung, eine Übertragungsvorrichtung und einen Magnetresonanztomographen bereitzustellen, die einfacher zu handhaben und kostengünstiger sind.

Die Aufgabe wird durch eine erfindungsgemäße Übertragungsvorrichtung nach Anspruch 1 sowie einen erfindungsgemäßen Magnetresonanztomographen nach Anspruch 8 gelöst.

Die erfindungsgemäße Übertragungsvorrichtung ist zum Übertragen eines Zwischenfrequenzsignals und eines Oszillatorsignals zum Erzeugen des Zwischenfrequenzsignals vorgesehen. Das Zwischenfrequenzsignal ist daher vorzugsweise in einem Frequenzbereich vorgesehen, der durch ein Mehrfaches der Magnetresonanzsignalbandbreite nach oben begrenzt ist, beispielsweise ein Doppeltes, Dreifaches, Fünffaches oder Zehnfaches der Magnetresonanzsignalbandbreite, also unterhalb von 2, 5, 10 oder 20 MHz ist. Dabei ist das Oszillatorsignal in einem Frequenzbereich um die Larmorfrequenz plus/minus der Zwischenfrequenz vorgesehen, vorzugsweise oberhalb von 50 MHz. Als zum Übertragen geeignet wird insbesondere eine Übertragungsvorrichtung angesehen, deren Dämpfung für die zu übertragenden Signale kleiner als 3, 6 oder 12 dB ist.

Die erfindungsgemäße Übertragungsvorrichtung weist eine symmetrische Übertragungsleitung zur Übertragung des Oszillatorsignals und des Zwischenfrequenzsignals auf. Eine symmetrische Übertragungsleitung ist eine Übertragungsleitung, bei der im Gegensatz zu einer asymmetrischen Übertragungsleitung nicht ein Leiter der mindestens zwei Leiter ein Bezugs- oder Massepotential aufweist. Bei einer symmetrischen Übertragungsleitung tragen beide Leiter eines Leiterpaares ein Signal, üblicherweise mit entgegengesetzter Polarität, sodass sich Abstrahlungen der Übertragungsleitungen bzw. Einstrahlungen in die Leitung aufheben. Symmetrische Übertragungsleitungen werden auch als differentielle Übertragungsleitungen bezeichnet. Beispielsweise kann es sich bei der symmetrischen Übertragungsleitung um eine Leitung mit verdrillten Adern-Paaren (twisted pair) oder Band- bzw. Stegleitung handeln.

Weiterhin weist die Übertragungseinrichtung ein Symmetrierglied (Balanced/Unbalanced, "BalUn") zur Anpassung einer unsymmetrischen Signalquelle und/oder Signalsenke an die symmetrische Übertragungsleitung auf, wobei das Symmetrierglied nur Ferrit-freie Induktivitäten aufweist. Ferrit-freie Induktivitäten sind beispielsweise Luftspulen, die keinen Ferritkern aufweisen, wie zum Beispiel einen Ringkern. Die erfindungsgemäße Übertragungsvorrichtung unterscheidet sich dadurch von Übertragungseinrichtungen, die ein breitbandiges Symmetrierglied mit einem Ringkerntransformator als BalUn aufweisen.

Die erfindungsgemäße Übertragungsvorrichtung kann zum einen auf vorteilhafte Weise in Magnetresonanzvorrichtungen in einem Untersuchungsbereich eingesetzt werden, da keine Ferrite Sättigungs-Effekte zeigen bzw. die statischen und dynamischen Magnetfelder stören. Darüber hinaus ist die erfindungsgemäße Übertragungsvorrichtung in der Lage, Signale von einer üblichen Lokalspule mit unsymmetrischem Signalausgang aufzunehmen und zu übertragen, auch wenn diese in unterschiedlichen Frequenzbereichen für Zwischenfrequenzsignale und Oszillatorsignale liegen.

Der erfindungsgemäße Magnetresonanztomograph teilt die Vorteile der erfindungsgemäßen Übertragungsvorrichtung.

Weitere vorteilhafte Ausführungsformen sind in den abhängigen Ansprüchen angegeben.

In einer möglichen Ausführungsform der erfindungsgemäßen Übertragungsvorrichtung ist die Übertragungsvorrichtung zum Übertragen des Oszillatorsignals mit einer Frequenz größer als 50 MHz und des Zwischenfrequenzsignals mit einer Frequenz kleiner als 20 MHz vorgesehen. Dabei ist die Bandbreite der beiden Signale vorzugsweise kleiner oder gleich 1, 2 oder 5 MHz. Vorzugsweise ist die Bandbreite zumindest des Zwischenfrequenzsignals größer oder gleich 500 kHz oder 1 MHz. Als zum Übertragen eines Frequenzbereichs geeignet wird die Übertragungsvorrichtung angesehene, wenn die Dämpfung in den zu übertragenden Frequenzbereichen kleiner als 3 dB, 6 dB oder 12 dB ist.

Auf vorteilhafte Weise ist die Übertragungsvorrichtung ausgelegt, Signale in zwei weit auseinanderliegenden Frequenzbereichen gleichermaßen gut zu übertragen, wie es bei einem Magnetresonanztomographen mit Zwischenfrequenz erforderlich ist.

In einer denkbaren Ausführungsform der erfindungsgemäßen Übertragungsvorrichtung weist die Übertragungsvorrichtung je ein lokales Dämpfungsminimum im Bereich der Frequenz des Oszillatorsignals und im Bereich der Frequenz des Zwischenfrequenzsignals auf. Als Bereich kann dabei die Bandbreite des jeweiligen Signals angesehen werden oder ein Vielfaches davon, beispielsweise das Doppelte, Dreifache oder Fünffache.

Eine breitbandige Anpassung einer unsymmetrischen Signalquelle an eine symmetrische Übertragungsquelle ist ohne Induktivitäten mit Ferriten (Ringkernen, klassischer BalUn) kaum möglich. Auf vorteilhafte Weise stellt die erfindungsgemäße Übertragungsvorrichtung eine Anpassung mit geringer Dämpfung in vorbestimmten, für die Funktion des Magnetresonanztomographen erforderlichen Frequenzbereichen bereit.

In einer möglichen Ausführungsform der erfindungsgemäßen Übertragungsvorrichtung weist die Übertragungsvorrichtung je ein lokales Maximum der Gleichtaktunterdrückung bei einem Frequenzbereich des Oszillatorsignals und bei einem Frequenzbereich des Zwischenfrequenzsignals auf. Als Frequenzbereich des Oszillatorsignals bzw. Zwischenfrequenzsignals wird dabei insbesondere ein Bereich von 1, 2, 5 oder 10 MHz um das jeweilige Signal angesehen.

Eine Gleichtaktunterdrückung gibt ein Maß für eine Unterdrückungen von Störungen an, die auf beide Adern einer symmetrischen Leitung einwirken. Ein Maximum der Gleichtaktunterdrückung im Bereich der Nutzsignale reduziert auf vorteilhafte Weise vor allem symmetrisch in die Leitung eingestrahlte Störungen (Gleichtakt-Störungen) und verbessert auf diese Weise die Bilderfassung.

In einer denkbaren Ausführungsform der erfindungsgemäßen Übertragungsvorrichtung weist diese als Symmetrierglied eine Boucherot-Brücke auf. Als Boucherot-Brücke wird eine aus mindestens zwei Boucherot-Gliedern, bzw. jeweils einem TiefpassGlied und einem Hochpass-Glied, bestehende Brückenschaltung bezeichnet. Die Boucherot-Brücke wandelt auf ihrer Bemessungsfrequenz ein unsymmetrisches Leitungssystem in ein symmetrisches Leitungssystem. Zudem kann mittels einer Boucherot-Brücke schmalbandig die Schnittstellenimpedanzen transformiert und somit Leistungsanpassung erzielt werden.

Eine Boucherot-Brücke ermöglicht auf ihrer Bemessungsfrequenz eine Anpassung von Hochfrequenzsignalen auch ohne breitbandige Ferrit-Spulen bzw. Transformatoren.

Die klassische Boucherot-Brücke weist den gravierenden Nachteil auf, dass sich die Funktion lediglich in einem einzigen und zudem relativ schmalen Frequenzband um die Bemessungsfrequenz herum nutzen lässt.

In einer möglichen Ausführungsform der erfindungsgemäßen Übertragungsvorrichtung weist die Boucherot-Brücke in mindestens einem oder auch in beiden Zweigen zwei Stufen auf. Als Stufe werden hierbei jeweils die Boucherot-Glieder d.h. das Hoch- und Tiefpassglied bezeichnet, die jeweils in einem Zweig der Boucherot-Brücke angeordnet sind. In der vorgeschlagenen Ausführungsform sind jeweils zwei Tiefpass-Glieder in einem Zweig der Boucherot-Brücke in Serie geschaltet und/oder zwei Hochpass-Glieder in dem anderen Zweig der Boucherot-Brücke. Dabei unterscheiden sich vorzugsweise die beiden jeweils in Serie geschalteten Boucherot-Glieder in den Werten ihrer Bauelemente, sodass sie unterschiedliche Impedanzkennlinien aufweisen.

Die Wechselwirkung der beiden Boucherot-Glieder mit unterschiedlicher Frequenz-Charakteristik in einem Zweig verbreitert auf vorteilhafte Weise die Bandbreite des Zweiges der Boucherot-Brücke.

In einer denkbaren Ausführungsform der erfindungsgemäßen Übertragungsvorrichtung weist die Boucherot-Brücke anstelle von ein oder mehrere Kapazitäten je einen Parallelresonanzkreis und/oder anstelle von ein oder mehreren Induktivitäten je einen Serienresonanzkreis auf. Vorzugsweise sind alle Kapazitäten der Boucherot-Brücke durch Parallelschwingkreise und alle Induktivitäten der Boucherot-Brücke durch Induktivitäten ersetzt.

Die Schwingkreise erlauben es auf vorteilhafte Weise durch ihre unterschiedlichen Resonanzfrequenzen das Symmetrierglied für unterschiedliche Frequenzbereiche gleichzeitig zu optimieren.

In einer Ausführungsform des erfindungsgemäßen Magnetresonanztomographen steht die Lokalspule über eine asymmetrische Schnittstelle in Signalverbindung mit der Übertragungsvorrichtung.

Der erfindungsgemäße Magnetresonanztomograph kann durch das Symmetrierglied der Übertragungsvorrichtung auf vorteilhafte Weise eine bisher verwendete Lokalspule mit Anschluss für ein Koaxialkabel in Verbindung mit der kostengünstigen Übertragungsvorrichtung verwenden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine beispielhafte schematische Darstellung eines erfindungsgemäßen Magnetresonanztomographen;
- Fig. 2: eine schematische Darstellung einer Ausführungsform der erfindungsbemäßen Übertragungsvorrichtung mit einer Lokalspule und einer Empfangseinheit;
- Fig. 3: eine schematische Darstellung einer Ausführungsform der erfindungsbemäßen Übertragungsvorrichtung mit einer Lokalspule und einer Empfangseinheit;
- Fig. 4: eine schematische Darstellung eines beispielhaften Symmetriergliedes.

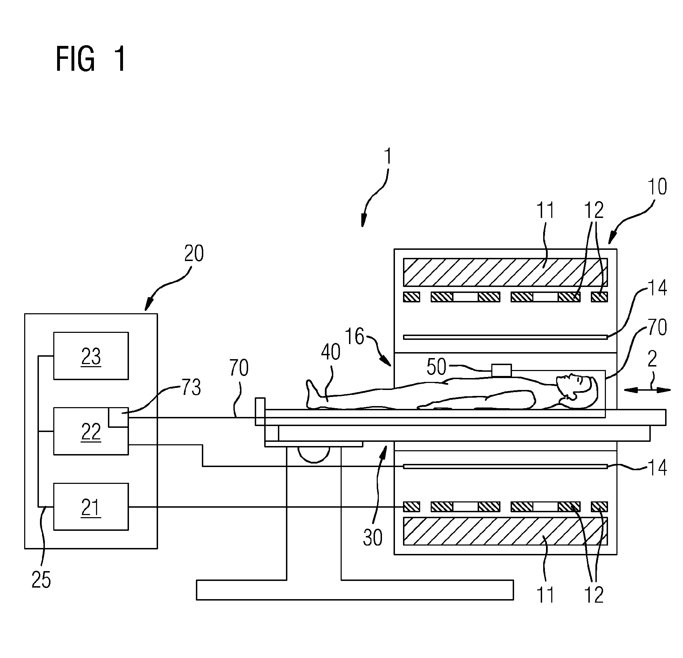

Fig. 1 zeigt eine schematische Darstellung eines erfindungsgemäßen Magnetresonanztomographen 1 mit einer erfindungsgemäßen Übertragungsvorrichtung 70.

Die Magneteinheit 10 weist einen Feldmagneten 11 auf, der ein statisches Magnetfeld B0 zur Ausrichtung von Kernspins von Proben bzw. in einem Körper eines Patienten 40 in einem Aufnahmebereich erzeugt. Der Aufnahmebereich ist in einem Patiententunnel 16 angeordnet, der sich in einer Längsrichtung 2 durch die Magneteinheit 10 erstreckt. Üblicherweise handelt es sich bei dem Feldmagneten 11 um einen supraleitenden Magneten, der magnetische Felder mit einer magnetischen Flussdichte von bis zu 3 Tesla, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 40 emittierte Resonanzsignale zu empfangen und über eine Signalleitung abzugeben. Weiterhin weist der erfindungsgemäße Magnetresonanztomograph eine oder mehrere Lokalspulen 50 auf, die in dem Patiententunnel 16 nahe am Patient 40 angeordnet sind.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Empfangseinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 40 zu erzeugen. Dabei können Pulsleistungen im Bereich von Kilowatt erreicht werden. Die einzelnen Einheiten sind über einen Signalbus 25 untereinander verbunden.

Die Lokalspule 50 empfängt vorzugsweise ein Magnetresonanzsignal aus dem Körper des Patienten 40, denn aufgrund des geringen Abstandes ist das Signal-zu-Rauschverhältnis (SNR) der Lokalspule 50 besser als bei einem Empfang durch die Körperspule 14. Das von der Lokalspule 50 empfangene MR-Signal wird in der Lokalspule 50 aufbereitet und mittels der erfindungsgemäßen Übertragungsvorrichtung 70 an die Empfangseinheit 22 des Magnetresonanztomographen 1 zur Auswertung und Bilderfassung weitergeleitet.

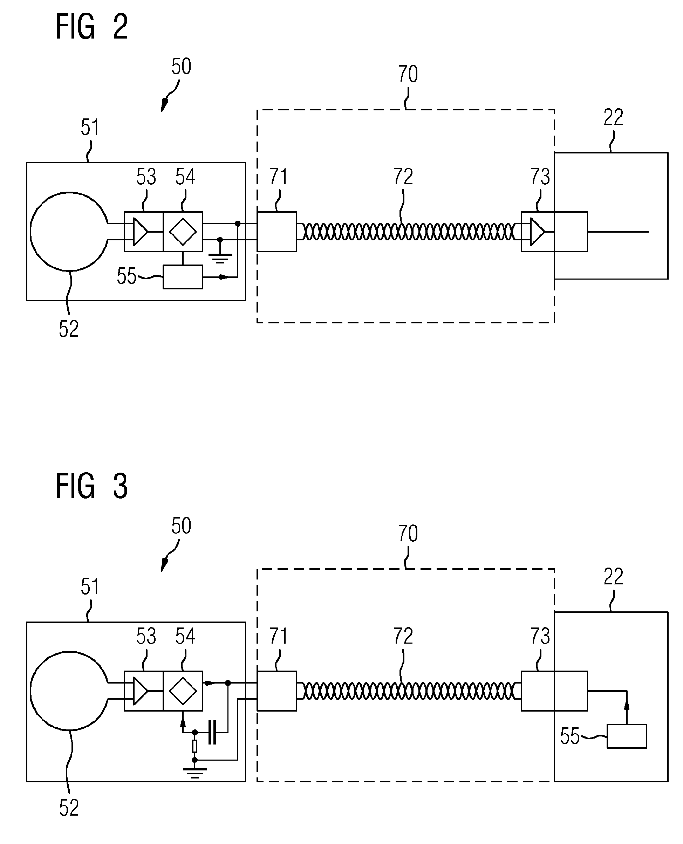

In Fig. 2 ist eine beispielhafte Ausführungsform der erfindungsbemäßen Übertragungsvorrichtung 70 mit der Lokalspule 50 und der Empfangseinheit 22 schematisch dargestellt. Die Lokalspule 50 weist in einem Gehäuse 51 eine Antennenspule 52 zum Empfang eines MR-Signals auf. Das empfangene MR-Signal wird anschließend von einem Vorverstärker 53 (low noise amplifier, LNA) verstärkt. Das MR-Signal liegt auf der Larmorfrequenz, die abhängig von der Stärke des Magnetfeldes B0 ist und bei 3 Tesla ca. 130 MHz beträgt. Die Bandbreite des MR-Signals beträgt hingegen lediglich ungefähr 0,5 MHz. Das MR-Signal wird deshalb in einem Mischer 54 mit einem Signal eines Oszillators 55 bzw. einem Oszillatorsignal gemischt und zur Übertragung auf eine niedrigere Frequenz zu einem Zwischenfrequenzsignal umgesetzt. Das Zwischenfrequenzsignal ist dabei wegen der einfacheren Schaltungstechnik üblicherweise ein asymmetrisches, auf eine Signalmasse bezogenes Signal.

Das Oszillatorsignal kann in einer Ausführungsform beispielsweise lokal in der Lokalspule 50 von einem Oszillator 55 erzeugt werden. Um die genaue Frequenz und die Phasenbeziehung des MR-Signals bei der Bilderfassung wieder herstellen zu können, wird dann vorzugsweise auch das Oszillatorsignal von der Lokalspule 50 zu der Empfangseinheit 22 übertragen, idealer Weise über eine gemeinsame Leitung bzw. Übertragungsvorrichtung 70.

Sind mehrere Lokalspulen 50 an einem Magnetresonanztomographen 1 vorgesehen, so ist es in einer Ausführungsform der Erfindung vorgesehen, dass der Oszillator 55 für mehrere Lokalspulen 50 gemeinsam das Oszillatorsignal bereitstellt und deshalb zentral in der Empfangseinheit 22 vorgesehen ist. Das Oszillatorsignal wird dann ebenfalls von der Übertragungsvorrichtung 70 zwischen Lokalspule 50 und Empfangseinheit 22 übertragen, jedoch in der anderen Richtung. Diese Ausführungsform ist in Fig. 3 dargestellt, die sich sonst jedoch nicht von der Fig. 2 unterscheidet. Die Trennung von Oszillatorsignal und Zwischenfrequenzsignal kann beispielsweise durch eine Hochpass/Tiefpass-Kombination (Diplexer) in der Lokalspule 50 erfolgen.

Die Übertragungsvorrichtung 70 weist zunächst ein Symmetrierglied 71 auf, das das asymmetrische Zwischenfrequenzsignal mit Massebezug in ein symmetrisches drittes Signal ohne Potentialbezug umsetzt. Zur Umsetzung der Signale mit unterschiedlicher Frequenz eignen sich zum Beispiel modifizierte Boucherot-Brücken, die nachfolgend dargestellt sind. Klassische Baluns mit Ringkernübertragern sind wegen der verwendeten Ferrite in der Umgebung des Feldmagneten 11 nicht geeignet.

Die Übertragung des dritten Signals erfolgt mittels einer symmetrischen Übertragungsleitung 72. Eine derartige Leitung kann beispielsweise ein Twisted-Pair Kabel sein, wie es bei der LAN-Verkabelung Verwendung findet, je nach Abschirmung und Eigenschaften beispielsweise als CAT4, CAT5 oder CAT6 bezeichnet. Es können auch die in der LAN-Verkabelungen üblichen Stecker wie RJ-45 Anwendung finden. Möglich sind aber auch andere symmetrische Leitungen wie Stegleitungen oder Strip-Leitungen auf flexiblen oder festen Platinen-Substraten. Ebenso sind andere Stecksysteme verwendbar.

In einer Ausführungsform ist es ebenfalls möglich, dass das Zwischenfrequenzsignal zunächst auf einer asymmetrischen Übertragungsleitung wie Koaxialkabel oder asymmetrische Streifenleitungen von der Lokalspule zu einem Übergabepunkt weggeführt wird. Ein möglicher Anwendungsfall kann beispielsweise eine Spine-Spule in einer Patientenliege 40 integriert sein, deren Zwischenfrequenzsignale bis zu einem Ende der Patientenliege 40 von einer asymmetrischen Übertragungsleitung auf einem Platinensubstrat geführt werden und anschließend erst von einem Symmetrierglied 71 für die weitere Übertragung auf einer symmetrischen Übertragungsleitung 72 zu einem dritten Signal angepasst werden. Die vorliegende Erfindung umfasst all diese denkbaren Kombinationen aus asymmetrischen und symmetrischen Übertragungsleitungen.

Vorzugsweise findet am Ende der symmetrischen Übertragungsleitung 72 wieder eine Umsetzung des dritten symmetrischen Signals in ein asymmetrisches Signal durch einen Umsetzer 73 statt, da die elektronische Signalverarbeitung in der Empfangseinheit 22 meist auf massebezogenen, asymmetrischen Signalen beruht. Überträgt die Übertragungsvorrichtung 70 das Oszillatorsignal und das Zwischenfrequenzsignal in die gleiche Richtung, kann der Umsetzer beispielsweise durch einen breitbandigen Differenzverstärker realisiert sein. Für eine Übertragung in beide Richtungen eignet sich ein Umsetzer 73 mit passiven Bauelementen, die beispielsweise wie in dem Symmetrierglied 71 mit einer oder mehreren Boucherot-Brücken realisiert sind.

Gemäß der Erfindung ist es auch denkbar, dass das Symmetrierglied 71 der Übertragungsvorrichtung 70 auch in dem Gehäuse 51 der Lokalspule angeordnet ist und/oder der Umsetzer 73 als Teil der Empfangseinheit 22 des Magnetresonanztomographen 1.

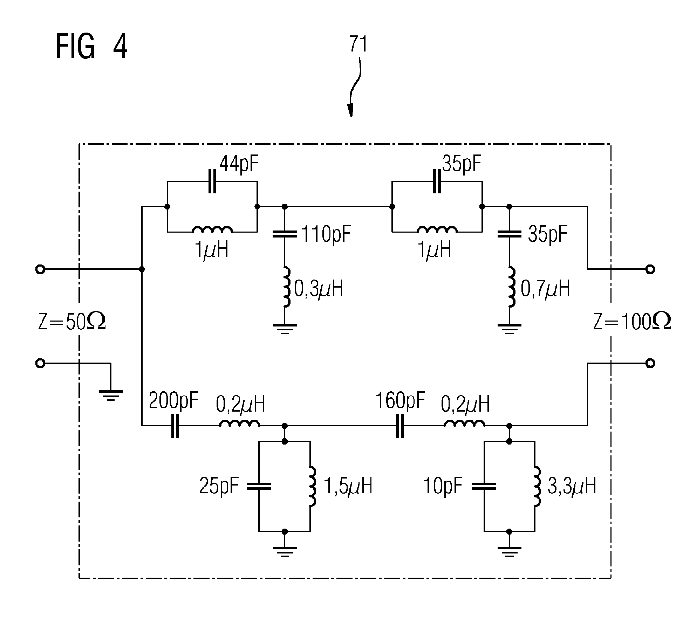

In Fig. 4 ist eine beispielhafte schematische Darstellung eines Symmetriegliedes 71 einer Ausführungsform der erfindungsgemäßen Übertragungsvorrichtung angegeben.

Das beispielhafte Symmetrierglied 71 in Fig. 4 führt eine Umsetzung von einem Signalport mit asymmetrischem Signal und einer Impedanz von 50 Ohm auf der linken Seite zu einem symmetrischen Signalport mit 100 Ohm Impedanz auf der rechten Seite. Durch die besondere Auslegung des dargestellten Symmetriergliedes 71 in Form einer "breitbandigen Dualband-Boucherot-Brücke" erfolgt die gewünschte Umsetzung sowohl in einem Frequenzbereich von 55 MHz bis 75 MHz für die Oszillatorfrequenz als auch in einem Frequenzbereich von 8 MHz bis 12 MHz für das Zwischenfrequenzsignal.

Eine einfachste Bauform einer Boucherot-Brücke weist einen asymmetrischen Signaleingang auf, der zwei Zweigen der Boucherot-Brücke zugeführt wird. Die Zweige entsprechen hier den Signalpfaden in der oberen bzw. unteren Hälfte des Schaltdiagramms in Fig. 4. Am Ausgang der jeweiligen Zweige liegt durch Phasenschiebung in den jeweiligen Zweigen in entgegengesetzter Richtung ein differentielles bzw. symmetrisches Ausgangssignal an. Wegen der passiven Bauelemente ist auch ein Signalfluss in umgekehrter Richtung denkbar, um ein symmetrisches Signal in ein asymmetrisches Signal umzusetzen. Die entgegengesetzte Phasenschiebung kann beispielsweise durch einen Hochpass in einem Zweig und einen Tiefpass in dem anderen Zweig erreicht werden. Der Hochpass und der Tiefpass werden allgemein auch als Boucherot-Glieder bezeichnet. In der Fig.4 sind zwei derartige Stufen in jedem Zweig in Serie geschaltet.

Darüber hinaus sind in der erfindungsgemäßen Ausführungsform der Fig. 4 jeweils die Kapazitäten in den jeweiligen Boucherot-Gliedern als Parallelschwingkreise ausgeführt und die Induktivitäten als Serienschwingkreis. Während Tief- und Hochpass jeweils nur eine Phasenverschiebung bis maximal 90 Grad im Betrag erzielen, erreichen Schwingkreise eine Phasenverschiebung von maximal 180 im Betrag, mit 90 Grad bei der Resonanzfrequenz. Durch geeignete Auslegung der Resonanzfrequenzen kann so ein Schwingkreis bei einer Frequenz als Tiefpass und bei einer anderen Frequenz als Hochpass agieren, sodass die Boucherot-Brücke mit den Schwingkreisen bei zwei weit unterschiedlichen Frequenzen als Symmetrierglied wirkt, unter Vertauschung der Wirkung der beiden Zweige.

Auf vorteilhafte Weise kann ein Symmetrierglied 71, das wie in Fig. 4 dargestellt, lediglich aus passiven und ferritfreien Bauelementen aufgebaut ist, in der Umgebung des Feldmagneten eingesetzt werden und ist darüber hinaus für beide Richtungen des Signalflusses geeignet, von dem symmetrischen zu dem unsymmetrischen Signalport und umgekehrt.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Übertragungsvorrichtung zum Übertragen eines Zwischenfrequenzsignals und eines Oszillatorsignals zum Heruntermischen des Zwischenfrequenzsignals, wobei die Übertragungsvorrichtung (70) aufweist:
eine symmetrische Übertragungsleitung (72) zur Übertragung des Oszillatorsignals und des Zwischenfrequenzsignals und
ein Symmetrierglied (71) zur Anpassung einer unsymmetrischen Signalquelle und/oder Signalsenke an die symmetrische Übertragungsleitung (72),
wobei das Symmetrierglied (71) nur Ferrit-freie Induktivitäten aufweist.

2. Übertragungsvorrichtung nach Anspruch 1, wobei das Oszillatorsignal eine Frequenz größer als 50 MHz und das Zwischenfrequenzsignal eine Frequenz kleiner als 20 MHz aufweist.

3. Übertragungsvorrichtung nach Anspruch 1 oder 2, wobei die Übertragungsvorrichtung (70) je ein lokales Dämpfungsminimum in einem Frequenzbereich des Oszillatorsignals und in einem Frequenzbereich des Zwischenfrequenzsignals aufweist.

4. Übertragungsvorrichtung nach einem der Ansprüche 1 bis 3, wobei die Übertragungsvorrichtung (70) je ein lokales Maximum der Gleichtaktunterdrückung in einem Frequenzbereich des Oszillatorsignals und in einem Frequenzbereich des Zwischenfrequenzsignals aufweist.

5. Übertragungsvorrichtung nach einem der vorhergehenden Ansprüche, wobei das Symmetrierglied (71) eine Boucherot-Brücke aufweist.

6. Übertragungsvorrichtung nach Anspruch 5, wobei die Boucherot-Brücke in mindestens einem Zweig der Boucherot-Brücke zwei Stufen aufweist.

7. Übertragungsvorrichtung nach Anspruch 5 oder 6, wobei ein oder mehrere Kapazitäten der Boucherot-Brücke durch je einen Parallelresonanzkreis ersetzt sind und/oder ein oder mehrere Induktivitäten durch je einen Serienresonanzkreis.

8. Magnetresonanztomograph, wobei der Magnetresonanztomograph (1) eine Übertragungsvorrichtung (70) nach einem der vorhergehenden Ansprüche, eine Lokalspule (50) und eine Empfangseinheit (22) aufweist, wobei die Lokalspule (50) mittels der Übertragungseinheit (22) in Signalverbindung mit der Empfangseinheit (22) zum Übertragen von Magnetresonanzsignalen steht.

9. Magnetresonanztomograph nach Anspruch 8, wobei die Lokalspule (50) über eine asymmetrische Schnittstelle in Signalverbindung mit der Übertragungsvorrichtung (70) steht.
